# EUROPEAN PATENT APPLICATION

(11) **EP 4 732 861 A1**
(43) Date of publication of application: **29.04.2026**
(21) Application number: 25210413.8
(22) Date of filing: 22.10.2025
(51) Int. Cl.: A61M 1/02, A61M 1/36, A61M 1/38, A61M 1/26

(54) **NOTIFICATION SYSTEM AND DEVICE FOR A BLOOD PROCEDURE**

(30) Priority: 23.10.2024 US 202463711112 P
(71) Applicant: Fenwal, Inc., Lake Zurich, IL 60047 (US)
(72) Inventor: MAHER, Jeffrey R., Lake Zurich, 60047 (US); QUEZADA, Francesca S., Lake Zurich, 60047 (US); DICOLA, Nicholas R., Lake Zurich, 60047 (US)
(74) Representative: Maikowski & Ninnemann Patentanwälte Partnerschaft mbB

(57) **Abstract**

Blood processing systems, devices, and methods including a durable hardware component, a disposable fluid flow circuit, and a controller configured to provide alerts and/or notifications. More particularly, blood processing systems and devices including a controller configured to monitor a blood processing procedure and to provide one or more notifications based on a monitored event during the blood processing procedure.

## Description

### Field of the Disclosure

The present disclosure is directed to systems and devices for blood processing procedures. More particularly, the present disclosure is directed to blood processing systems and devices configured to provide alerts and/or notifications to an operator during the blood processing procedure.

### Background

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a human donor because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

Existing apheresis devices typically include various sensors and monitoring systems to track the progress of the procedure and ensure the safety and efficacy of the blood separation process. For example, many apheresis devices monitor the flow rate, pressure, and composition of the blood as it passes through the devices. If any of these parameters deviate outside of pre-defined, hard-coded operating ranges, the device may generate alerts or notifications to warn the operator.

These hard-coded notification thresholds are typically set by the device manufacturer based on general guidelines and recommendations, rather than being dynamically adjusted based on the specific patient or donor characteristics, procedure type, or real-time trends in the monitored data. As a result, the notification system may not always provide the most relevant or timely alerts to the operator.

For example, a limitation of hard-coded alert thresholds is the ability to notify an operator when updates to procedure targets or donor parameters may be necessary. Such changes can significantly impact the target whole blood volume to process and the targeted procedure products and volumes. Near the end of a procedure, changes may no longer be feasible without negative consequences, such as instability in the collection process due to changes in processing rates, large volumes of blood returned to the donor in a short time, large volumes required to be collected in a short time, and/or exceeding donor safety limits, thus requiring immediate procedure termination.

Additionally, traditional apheresis devices may be configured to notify an operator when the remaining whole blood volume to process reaches a hard-coded threshold, when a hard-coded minimum remaining solution volume is reached, and/or when the procedure is nearing completion based on a hard-coded amount of target blood product collected. Because procedures may include variable processing rates, the alerts may occur at varying times between procedures. This variability diminishes the effectiveness of an alert because an operator may not realize how much time is actually remaining in the step or overall procedure.

For example, if the pre-defined remaining whole blood threshold is 500 ml and the system is processing at 50 ml/min, the operator is notified with 10 minutes remaining, but if the rate is 100 ml/min, the notification comes with only 5 minutes remaining.

Thus, there is a need for improved notification systems associated with apheresis systems and devices.

### Summary

In one aspect, a blood processing system is provided. The blood processing system includes a reusable separation device, wherein the separation device includes a separator and a disposable fluid circuit including a separation chamber and a plurality of containers. The disposable fluid circuit is configured to be associated with the reusable separation device. The reusable separation device includes a controller configured to monitor a blood processing procedure and to provide one or more notifications based on a monitored event during the blood processing procedure.

In another aspect, a blood processing device is provided. The blood processing device includes a separator and a controller configured to monitor a blood processing procedure and to provide one or more notifications based on a monitored event during the blood processing procedure.

### Brief Description of the Drawings

FIG. 1 is a perspective view of an exemplary fluid processing device.
FIG. 2 is a schematic view of an exemplary disposable fluid flow circuit that may be mounted to the fluid processing device of FIG. 1 to complete a fluid processing system according to an aspect of the present disclosure.
FIG. 3 is a top plan view of an exemplary cassette of the fluid flow circuit of FIG. 2, which can be actuated to perform a variety of different fluid processing procedures in association with the fluid processing device shown in FIG. 1.
FIG. 4 is a schematic view of the fluid flow circuit of FIG. 2 mounted on the fluid processing device of FIG. 1, showing the system carrying out a fluid processing procedure.

### Detailed Description of the Embodiments

A more detailed description of the systems and methods in accordance with the present disclosure is set forth below. It will be understood that the description below of specific devices and methods is intended to be exemplary, and not exhaustive of all possible variations or applications.

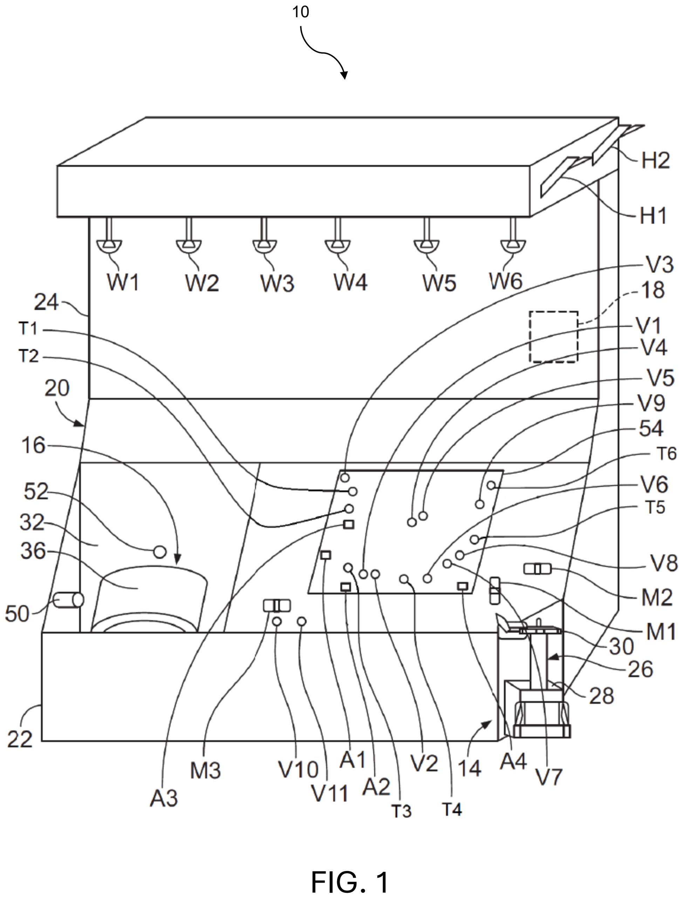

FIGS. 1-4 show components of a fluid processing system that embody various aspects of the present subject matter. Use of the system for separating blood into two or more components and collecting at least one of the components will be described herein, though it should be understood that systems according to the present disclosure can be used for processing a variety of fluids, which may include bodily fluids and non-bodily fluids.

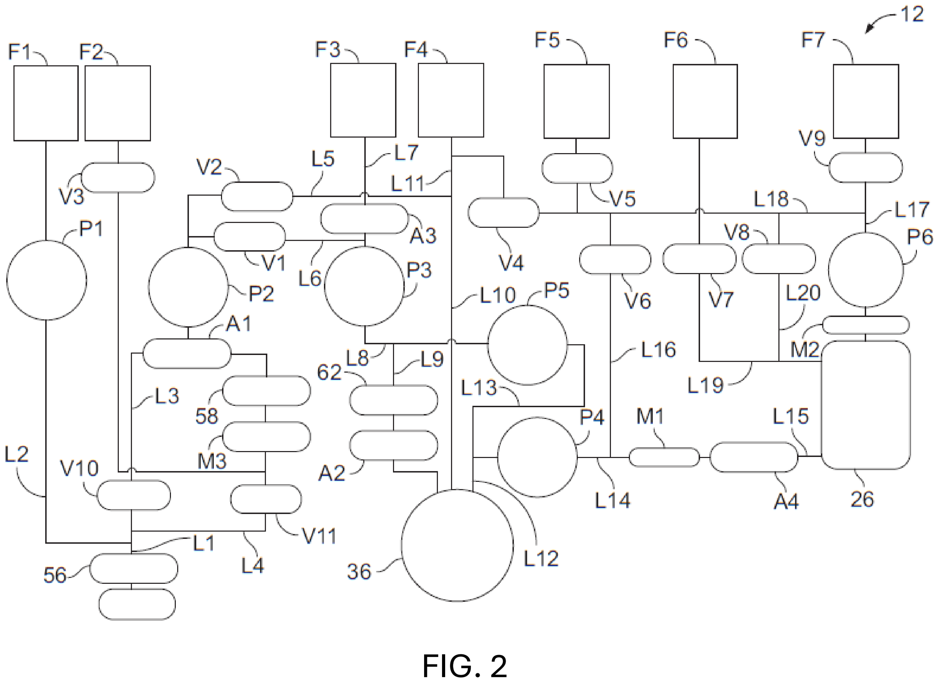

In one embodiment, the system includes two principal components, a durable and reusable fluid processing device 10 (FIG. 1) and a disposable fluid flow circuit 12 (FIG. 2). The illustrated fluid processing device 10 includes a spinning membrane separator drive unit 14, a centrifuge or centrifugal separator 16, additional components that control fluid flow through the disposable flow circuit 12, and a controller 18, which governs the operation of the other components of the fluid processing device 10 to perform a fluid processing procedure. A fluid processing device 10 of the type shown in FIG. 1 is described in greater detail in PCT Patent Application Publication No. WO 2018/053217 A1, which is hereby incorporated herein by reference. While the principles described herein may be employed when using the fluid processing device 10 of FIG. 1, it should be understood that these same principles may be applied to other fluid processing devices, including devices employing single separation technologies or approaches.

### I. The Durable Fluid Processing Device

The fluid processing device 10 (FIG. 1) is configured as a durable item that is capable of long-term use. It should be understood that the fluid processing device 10 of FIG. 1 is merely exemplary of one possible configuration and that fluid processing devices according to the present disclosure may be differently configured.

In the illustrated embodiment, the fluid processing device 10 is embodied in a single housing or case 20. The illustrated case 20 includes a generally horizontal portion 22 (which may include an inclined or angled face or upper surface for enhanced visibility and ergonomics) and a generally vertical portion 24. The spinning membrane separator drive unit 14 and the centrifugal separator 16 are shown as being incorporated into the generally horizontal portion 22 of the case 20, while the controller 18 is shown as being incorporated into the generally vertical portion 24.

### A. Spinning Membrane Separator Drive Unit

The fluid processing device 10 includes a spinner support or spinning membrane separator drive unit 14 for accommodating a generally cylindrical spinning membrane separator 26 of the fluid flow circuit 12. U.S. Patent No. 5,194,145 (which is hereby incorporated herein by reference) describes an exemplary spinning membrane separator drive unit that would be suitable for incorporation into the fluid processing device 10, but it should be understood that the spinning membrane separator drive unit 14 may be differently configured without departing from the scope of the present disclosure.

The illustrated spinning membrane separator drive unit 14 has a base 28 configured to receive a lower portion of the spinning membrane separator 26 and an upper end cap 30 to receive an upper portion of the spinning membrane separator 26. Preferably, the upper end cap 30 is positioned directly above the base 28 to orient a spinning membrane separator 26 received by the spinning membrane separator drive unit 14 vertically and to define a vertical axis about which the spinning membrane separator 26 is spun. While it may be advantageous for the spinning membrane separator drive unit 14 to vertically orient a spinning membrane separator 26, it is also within the scope of the present disclosure for the spinning membrane separator 26 to be differently oriented when mounted to the fluid processing device 10.

In one embodiment, one of the base 28 and upper end cap 30 of the spinning membrane separator drive unit 14 is movable with respect to the other, which may allow differently sized spinning membrane separators 26 to be received by the spinning membrane separator drive unit 14. For example, the upper end cap 30 may be translated vertically with respect to the base 28 and locked in a plurality of different positions, with each locking position corresponding to a differently sized spinning membrane separator 26.

At least one of the base 28 and the upper end cap 30 is configured to spin one or more components of the spinning membrane separator 26 about the axis defined by the spinning membrane separator drive unit 14. The mechanism by which the spinning membrane separator drive unit 14 spins one or more components of the spinning membrane separator 26 may vary without departing from the scope of the present disclosure. In one embodiment, a component of the spinning membrane separator 26 to be spun includes at least one element configured to be acted upon by a magnet (*e.g*., a metallic material), while the spinning membrane separator drive unit 14 includes a magnet (*e.g*., a series of magnetic coils or semi-circular arcs). By modulating the magnetic field acting upon the aforementioned element of the spinning membrane separator 26, the component or components of the spinning membrane separator 26 may be made to spin in different directions and at varying speeds. In other embodiments, different mechanisms may be employed to spin the component or components of the spinning membrane separator 26.

Regardless of the mechanism by which the spinning membrane separator drive unit 14 spins the component or components of the spinning membrane separator 26, the component or components of the spinning membrane separator 26 is/are preferably spun at a speed that is sufficient to create Taylor vortices in a gap between the spinning component and a stationary component of the spinning membrane separator 26 (or a component that spins at a different speed). Fluid to be separated within the spinning membrane separator 26 flows through this gap, and filtration may be dramatically improved by the creation of Taylor vortices.

### B. Centrifugal Separator

As for the centrifugal separator 16, it includes a centrifuge compartment 32 that receives a centrifugal separation chamber 36 of the fluid flow circuit 12, as well as other components of the centrifugal separator 16. Further details as to the configuration and operation of an exemplary centrifugal separator are set forth in PCT Patent Application Publication No. WO 2018/053217 A1.

Fluid (*e.g*., anticoagulated whole blood) is introduced into the centrifugal separation chamber 36 by an umbilicus, with the fluid being separated into a layer of less dense components (*e.g*., platelet-rich plasma, in the case of blood being separated) and a layer of more dense components (*e.g*., packed red blood cells) within the centrifugal separation chamber 36 as a result of centrifugal forces as it rotates. Components of an interface monitoring system may be positioned within the centrifuge compartment 32 to oversee separation of fluid within the centrifugal separation chamber 36. The interface monitoring system may include a light source 50 and a light detector 52, which is positioned and oriented to receive at least a portion of the light emitted by the light source 50.

The orientation of the various components of the interface monitoring system depends at least in part on the particular configuration of the centrifugal separation chamber 36. In general, though, the light source 50 emits a light beam (*e.g*., a laser light beam) through the separated fluid components within the centrifugal separation chamber 36 (which may be formed of a material that substantially transmits the light or at least a particular wavelength of the light without absorbing it). A portion of the light reaches the light detector 52, which transmits a signal to the controller 18 that is indicative of the location of an interface between the separated fluid components. If the controller 18 determines that the interface is in the wrong location (which can affect the separation efficiency of the centrifugal separator 16 and/or the quality of the separated fluid components), then it can issue commands to the appropriate components of the fluid processing device 10 to modify their operation so as to move the interface to the proper location.

### C. Other Components Of The Fluid Processing Device

In addition to the spinning membrane separator drive unit 14 and the centrifugal separator 16, the fluid processing device 10 may include other components compactly arranged to aid fluid processing.

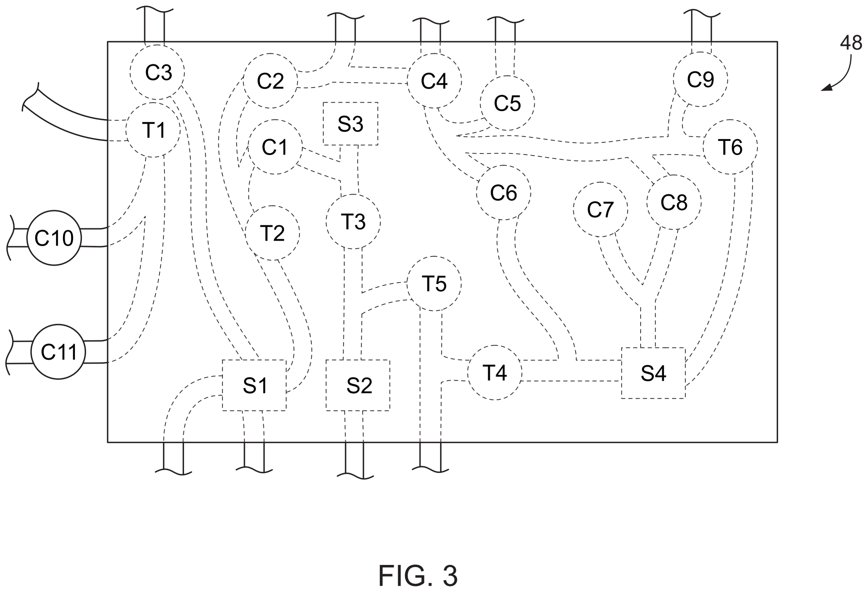

The generally horizontal portion 22 of the case 20 of the illustrated fluid processing device 10 includes a cassette station 54, which accommodates a flow control cassette 48 of the fluid flow circuit 12. An exemplary flow control cassette 48 (which will be described in greater detail herein) is shown in FIG. 3. In one embodiment, the cassette station 54 is similarly configured to the pneumatic-actuated pump and valve station described in U.S. Patent Application Publication No. 2006/0161092. The illustrated cassette station 54 includes a plurality of clamps or valves V1-V9 (FIG. 1), which move between a plurality of positions (*e.g*., between a retracted or lowered position and an actuated or raised position) to selectively contact or otherwise interact with corresponding valve stations C1-C9 of the flow control cassette 48 of the fluid flow circuit 12 (FIG. 3). Depending on the configuration of the fluid flow circuit 12, its cassette 48 may not include a valve station C1-C9 for each valve V1-V9 of the cassette station 54, in which case fewer than all of the valves V1-V9 will be used in a fluid processing procedure.

In the actuated position, a valve V1-V9 engages the associated valve station C1-C9 to prevent fluid flow through that valve station C1-C9 (*e.g*., by closing one or more ports associated with the valve station C1-C9, thereby preventing fluid flow through that port or ports). In the retracted position, a valve V1-V9 is disengaged from the associated valve station C1-C9 (or less forcefully contacts the associated valve station C1-C9 than when in the actuated position) to allow fluid flow through that valve station C1-C9 (*e.g*., by opening one or more ports associated with the valve station C1-C9, thereby allowing fluid flow through that port or ports). Additional clamps or valves V10 and V11 may be positioned outside of the cassette station 54 to interact with portions of valve stations C10 and C11 (which may be lengths of tubing) of the fluid flow circuit 12 to selectively allow and prevent fluid flow therethrough. The valves V1-V9 and corresponding valve stations C1-C9 of the cassette station 54 and cassette 48 may be differently configured and operate differently from the valves V10 and V11 and the valve stations C10 and C11 that are spaced away from the cassette station 54.

The cassette station 54 may be provided with additional components, such as pressure sensors A1-A4, which interact with sensor stations S1-S4 of the cassette 48 to monitor the pressure at various locations of the fluid flow circuit 12. For example, if the fluid source is a human donor, one or more of the pressure sensors A1-A4 may be configured to monitor the pressure of the donor's vein during blood draw and return. Other pressure sensors A1-A4 may monitor the pressure of the spinning membrane separator 26 and the centrifugal separation chamber 36. The controller 18 may receive signals from the pressure sensors A1-A4 that are indicative of the pressure within the fluid flow circuit 12 and, if a signal indicates a low- or high-pressure condition, the controller 18 may initiate an alarm or error condition to alert an operator to the condition and/or to attempt to bring the pressure to an acceptable level without operator intervention.

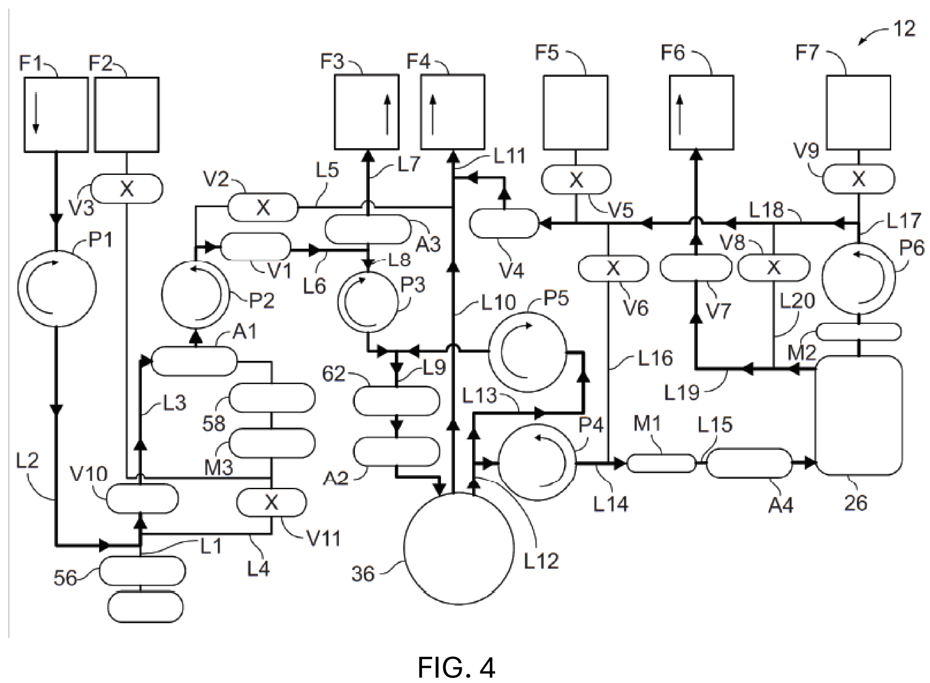

The fluid processing device 10 may also include a plurality of pumps P1-P6 (which may be collectively referred to as a pump assembly or pump system) to cause fluid to flow through the fluid flow circuit 12. The pumps P1-P6 may be differently or similarly configured and/or function similarly or differently from each other. In the illustrated embodiment, the pumps P1-P6 are configured as pneumatic pumps, which may be generally configured as described in U.S. Patent Application Publication No. 2006/0161092. An actuator of each pump P1-P6 is controlled by the controller 18 to alternately apply positive and negative pressures to a flexible membrane or diaphragm 64 of a different pump chamber T1-T6 defined by the flow control cassette 48 (Fig. 4) so as to cause fluid to flow through a portion of the fluid flow circuit 12. The configurations and operation of the pump system and the controller 18 will be described in greater detail herein.

The illustrated fluid processing device 10 also includes a spinner inlet sensor M1 for determining one or more properties of a fluid flowing into a spinning membrane separator 26 mounted within the spinning membrane separator drive unit 14. If the fluid flowing into the spinning membrane separator 26 is whole blood (which may include anticoagulated whole blood), the spinner inlet sensor M1 may be configured to determine the hematocrit of the blood flowing into the spinning membrane separator 26. If the fluid flowing into the spinning membrane separator 26 is platelet-rich plasma, the spinner inlet sensor M1 may be configured to determine the platelet concentration of platelet-rich plasma flowing into the spinning membrane separator 26. The spinner inlet sensor M1 may detect the one or more properties of a fluid by optically monitoring the fluid as it flows through tubing of the fluid flow circuit 12, or by any other suitable approach. The controller 18 may receive signals from the spinner inlet sensor M1 that are indicative of the one or more properties of fluid flowing into the spinning membrane separator 26 and use the signals to optimize the fluid processing procedure based upon that property or properties. If the property or properties is/are outside of an acceptable range, then the controller 18 may initiate an alarm or error condition to alert an operator to the condition. A suitable device and method for monitoring hematocrit and/or platelet concentration is described in U.S. Patent No. 6,419,822 (which is hereby incorporated herein by reference), but it should be understood that a different approach may also be employed for monitoring one or more properties of a fluid or fluid component flowing into the spinning membrane separator 26.

The illustrated fluid processing device 10 further includes a spinner outlet sensor M2, which accommodates tubing of the fluid flow circuit 12 that flows a separated fluid component out of the spinning membrane separator 26. The spinner outlet sensor M2 monitors the separated fluid component to determine one or more properties thereof, and may do so by optically monitoring the separated fluid component as it flows through the tubing or by any other suitable approach. In one embodiment, separated plasma flows through the tubing, in which case the spinner outlet sensor M2 may be configured to determine the amount of cellular blood components in the plasma and/or whether the plasma is hemolytic and/or lipemic. This may be done using an optical monitor of the type described in U.S. Patent No. 8,556,793 (which is hereby incorporated herein by reference) that measures the optical density of the fluid in the associated tubing, or by any other suitable device and/or method.

The illustrated fluid processing device also includes an air detector M3 (*e.g*., an ultrasonic bubble detector), which accommodates tubing of the fluid flow circuit 12 that flows fluid to a recipient. It may be advantageous to prevent air from reaching the recipient, whether a human recipient (*e.g*., the same human that serves as the blood source) or a non-human recipient (*e.g*., a storage bag or container), so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (*e.g*., by reversing the flow of fluid through the tubing or diverting flow to a vent location).

The generally vertical portion 24 of the case 20 may include a plurality of volume measurement systems W1-W6 (six are shown, but more or fewer may be provided), each configured to be associated with one or more fluid containers F1-F7 of the fluid flow circuit 12 (FIGS. 2 and 4). Each volume measurement system W1-W6 is configured to work in combination with the controller 18 to measure a current volume of fluid within an associated fluid container F1-F7 and to calculate a change in that volume between two or more points in time. The individual volume measurement systems W1-W6 may be variously configured without departing from the scope of the present disclosure, which may include two or more of the volume measurement systems W1-W6 being differently configured. In one exemplary embodiment, a volume measurement system W1-W6 may be configured as or include a weight scale configured to support and measure the weight of a fluid within an associated fluid container F1-F7 (with the measured weight being converted to a volume by a component of the volume measurement system W1-W6 or by the controller 18). In another exemplary embodiment, a volume measurement system W1-W6 may include one or more sensors configured to detect a volume and/or a change in volume of a fluid within an associated fluid container F1-F7. Volume measurement systems including additional components (*e.g*., both a weight scale and a sensor) and/or alternative components may also be employed without departing from the scope of the present disclosure.

Regardless of its particular configuration, each volume measurement system W1-W6 may transmit to the controller 18 a signal that is indicative of the volume of the fluid within the associated container F1-F7 to track the change of volume during the course of a procedure. This allows the controller 18 to process the incremental volume changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated case 20 is also provided with a plurality of hooks or supports H1 and H2 that may support various components of the fluid flow circuit 12 or other suitably sized and configured objects.

### D. Controller

According to an aspect of the present disclosure, the fluid processing device 10 includes a controller 18, which is suitably configured and/or programmed to control operation of the fluid processing device 10. In one embodiment, the controller 18 comprises a main processing unit (MPU), which can comprise, e.g., a Pentium^{™} type microprocessor made by Intel Corporation, although other types of conventional microprocessors can be used. In one embodiment, the controller 18 may be mounted inside the generally vertical portion 24 of the case 20, adjacent to or incorporated into an operator interface station (e.g., a touchscreen). In other embodiments, the controller 18 and operator interface station may be associated with the generally horizontal portion 22 or may be incorporated into a separate device that is connected (either physically, by a cable or the like, or wirelessly) to the fluid processing device 10.

The controller 18 is configured and/or programmed to execute at least one fluid processing procedure but, more advantageously, is configured and/or programmed to execute a variety of different fluid processing procedures. For example, the controller 18 may be configured and/or programmed to carry out one or more of the following: a double unit red blood cell collection procedure, a plasma collection procedure, a plasma/red blood cell collection procedure, a red blood cell/platelet/plasma collection procedure, a platelet collection procedure, and a platelet/plasma collection procedure.

More particularly, in carrying out these fluid processing procedures, the controller 18 is configured and/or programmed to control one or more of the following tasks: drawing fluid into a fluid flow circuit 12 mounted to the fluid processing device 10, conveying fluid through the fluid flow circuit 12 to a location for separation (i.e., into a spinning membrane separator 26 or centrifugal separation chamber 36 of the fluid flow circuit 12), separating the fluid into two or more components as desired, and conveying the separated components into storage containers, to a second location for further separation (*e.g*., into whichever of the spinning membrane separator 26 and centrifugal separation chamber 36 that was not used in the initial separation stage), or to a recipient (which may be a source from which the fluid was originally drawn).

This may include instructing the spinning membrane separator drive unit 14 and/or the centrifugal separator 16 to operate at a particular rotational speed and instructing a pump P1-P6 to apply a particular (positive or negative) pressure to a flexible membrane or diaphragm 64 of an associated pump chamber T1-T6 of the cassette 48 for a particular duration to convey fluid through a portion of the fluid flow circuit 12 at a particular flow rate. Hence, while it may be described herein that a particular component of the fluid processing device 10 (*e.g.*, the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the fluid processing device 10 (*e.g*., the pressure sensors A1-A4) to monitor various aspects of the operation of the fluid processing device 10 and characteristics of the fluid and separated fluid components as they flow through the fluid flow circuit 12. If the operation of any of the components and/or one or more characteristics of the fluid or separated fluid components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

For example, the controller 18 may include an interface control module, which receives signals from the light detector 52 of the interface monitoring system. The signals that the controller 18 receives from the light detector 52 are indicative of the location of an interface between the separated fluid components within the centrifugal separation chamber 36. If the controller 18 determines that the interface is in the wrong location, then it can issue commands to the appropriate components of the fluid processing device 10 to modify their operation so as to move the interface to the proper location. For example, the controller 18 may instruct one of the pumps P1-P6 to cause fluid to flow into the centrifugal separation chamber 36 at a different rate and/or for a separated fluid component to be removed from the centrifugal separation chamber 36 at a different rate and/or for the centrifugal separation chamber 36 to be spun at a different speed by the centrifugal separator 16.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

### II. The Disposable Fluid Flow Circuit

As for the fluid flow circuit or flow set 12 (FIG. 2), it is intended to be a sterile, single use, disposable item. Before beginning a given fluid processing procedure, the operator mounts various components of the fluid flow circuit 12 to the case 20 in association with the fluid processing device 10. The controller 18 implements the procedure based upon preset protocols, taking into account other input from the operator. Upon completing the procedure, the operator removes the fluid flow circuit 12 from association with the fluid processing device 10. The portions of the fluid flow circuit 12 holding the collected fluid component or components (*e.g.*, collection containers or bags) are removed from the case 20 and retained for storage, transfusion, or further processing. The remainder of the fluid flow circuit 12 is removed from the case 20 and discarded.

In the illustrated embodiment, the fluid flow circuit 12 includes a cassette 48 (FIG. 3), to which the other components of the fluid flow circuit 12 are connected by flexible tubing. The other components may include a plurality of fluid containers F1-F7. In the context of the present disclosure, these containers include an anticoagulant container F1, a saline container F2, an in-process container F3, a return container F4, a plasma collection container F5, a platelet collection container F6, and an (optional) additive container F7. The illustrated flow circuit 12 further includes one or more fluid source access devices (*e.g*., a connector for accessing blood within a fluid container or a phlebotomy needle), a spinning membrane separator 26 and a centrifugal separation chamber 36.

The flow control cassette 48 provides a centralized, programmable, integrated platform for all the pumping and many of the valving functions required for a given fluid processing procedure. In one embodiment, the cassette 48 is similarly configured to the cassette of U.S. Patent Application Publication No. 2006/0161092, but is adapted to include the various stations and flow paths required to carry out the fluid processing procedures implemented by the fluid processing system.

In use, the cassette 48 is mounted to the cassette station 54 of the fluid processing device 10, with a flexible membrane or diaphragm 64 of the cassette 48 placed into contact with the cassette station 54. The flexible diaphragm 64 overlays an array of interior cavities formed by the body of the cassette 48. The different interior cavities define sensor stations S1-S4, valve stations C1-C9, pump stations T1-T6, and a plurality of flow paths. The side of the cassette 48 opposite the flexible diaphragm 64 may be sealed by another flexible diaphragm or by a rigid cover, thereby sealing fluid flow through the cassette 48 from the outside environment.

Each sensor station S1-S4 is aligned with an associated pressure sensor A1-A4 of the cassette station 54, with each pressure sensor A1-A4 being capable of monitoring the pressure within the associated sensor station S1-S4. Each valve station C1-C9 is aligned with an associated valve V1-V9 and may define one or more ports that allow for fluid communication between the valve station C1-C9 and another interior cavity of the cassette 48 (*e.g*., a flow path). As described above, each valve V1-V9 is movable under command of the controller 18 to move between a plurality of positions (*e.g*., between a retracted or lowered position and an actuated or raised position) to selectively contact the valve stations C1-C9 of the cassette 48. In the actuated position, a valve V1-V9 engages the associated valve station C1-C9 to close one or more of its ports to prevent fluid flow therethrough. In the retracted position, a valve V1-V9 is disengaged from the associated valve station C1-C9 (or less forcefully contacts the associated valve station C1-C9 than when in the actuated position) to open one or more ports associated with the valve station C1-C9, thereby allowing fluid flow therethrough.

As described above, the cassette 48 defines a plurality of pump chambers T1-T6, with each pump chamber interacting with a different one of the pneumatic pumps P1-P6 of the cassette station 54 of the fluid processing device 10. The different pumps P1-P6 may interact with the pump stations T1-T6 of the cassette 48 to perform different tasks during a procedure, but in the context of the present disclosure, a different one of the pumps P1-P6 may be configured to serve as an anticoagulant pump P1, a source pump P2, a centrifuge pump P3, an outlet pump P4, a recirculation pump P5, and a plasma pump P6.

Various lengths of tubing extend from the side surface of the cassette 48 to connect to the other components of the fluid flow circuit 12, such as the various fluid containers F1-F7, the spinning membrane separator 26, and the centrifugal separation chamber 36. The tubing connected to the centrifugal separator chamber 36 (which includes one inlet tube and two outlet tubes) may be aggregated into an umbilicus.

Various additional components may be incorporated into the tubing leading out of the cassette 48 or into one of the cavities of the cassette 48. For example, as shown in FIG. 2, a manual clamp 56 may be associated with a line or lines leading to the fluid source, a return line filter 58 (*e.g*., a microaggregate filter) may be associated with a line leading to a fluid recipient, and/or an air trap 62 may be positioned on a line upstream of the centrifugal separation chamber 36.

### III. Exemplary Fluid Processing Procedure

An exemplary fluid processing procedure according to the present disclosure will now be described. In the exemplary procedure, blood is separated via centrifugation into packed red blood cells and platelet-rich plasma, with a portion of the platelet-rich plasma being recirculated back through a centrifugal separation chamber and another portion being separated into platelet concentrate and platelet-poor plasma until a target volume of platelets has been collected. It should be understood that the below-described procedure is merely exemplary and that the principles described herein may be practiced in combination with other fluid processing procedures (*e.g*., procedures in which platelet-poor plasma is recirculated through a centrifugal separation chamber during blood separation) without departing from the scope of the present disclosure.

Prior to processing, an operator selects the desired protocol (*e.g*., using an operator interface station, if provided), which informs the controller 18 of the manner in which it is to control the other components of the fluid processing device 10 during the procedure. This may include first selecting one of a plurality of possible procedures that the system is capable of executing and then, after selecting the nature of the procedure, selecting one or more parameters to be in effect during the procedure. For example, this may include selecting a platelet collection procedure from among a variety of blood separation procedures and then selecting a total volume of blood to be processed or a target volume of platelets to be collected during the procedure. If the fluid source is a living source (*e.g*., a donor or patient), the operator may proceed to enter various parameters, such as the sex/height/weight of the source. In one embodiment, the operator may also enter one or more characteristics of the fluid to be processed, such as a platelet pre-count.

If there are any fluid containers (*e.g*., a platelet additive solution container) that are not integrally formed with the fluid flow circuit 12, they may be connected to the fluid flow circuit 12 (*e.g*., by piercing a septum of a tube of the fluid flow circuit 12 or via a luer connector), with the fluid flow circuit 12 then being mounted to the fluid processing device 10 (including the fluid containers F1-F7 being associated with the volume measurement systems W1-W6, as appropriate). In one exemplary embodiment, each volume measurement system W1-W6 includes a weight scale associated with a hook from which a fluid container may be hung. In another exemplary embodiment, at least one of the volume measurement systems W1-W6 includes a weight scale associated with a horizontal platform or surface, with a container being placed onto the platform or surface for support while the weight scale sends signals indicative of the weight of the container (and its contents) to be sent to the controller 18 throughout the course of a procedure. In other embodiments, a fluid container may be associated with a volume measurement system omitting a weight scale, but including other means for measuring the volume of fluid within the container (*e.g*., one or more sensors).

An integrity check of the fluid flow circuit 12 may be executed by the controller 18 to ensure that the various components are properly connected and functioning. Following a successful integrity check, the fluid source is connected to the fluid flow circuit 12 (*e.g*., by connecting to a container of previously collected fluid or by phlebotomizing a donor), and the fluid flow circuit 12 may be primed (*e.g*., using saline pumped from a saline container F2 by operation of one or more of the pumps P1-P6 of the fluid processing device 10).

After the fluid flow circuit 12 has been primed, fluid processing may begin. In a first phase of an exemplary platelet collection procedure (FIG. 4), blood is drawn into the fluid flow circuit 12 from a blood source. If the blood source is a donor, then blood may be drawn into the fluid flow circuit 12 through a single needle that is connected to the cassette by line L1. Line L1 may include a manual clamp 56 that may initially be in a closed position to prevent fluid flow through line L1. When processing is to begin, an operator may move the manual clamp 56 from its closed position to an open position to allow fluid flow through line L1.

The blood is drawn into line L1 by the source pump P2 of the fluid processing device 10. Anticoagulant from the anticoagulant container F1 may be drawn through line L2 under action of the anticoagulant pump P1 and added to the blood at a junction of lines L1 and L2.

In the illustrated embodiment, valve V10 is open to allow anticoagulated blood to flow through line L3 and a cassette sensor station associated with pressure sensor A1, while valve V11 is closed to prevent fluid flow through line L4. If the blood source is a living body (*e.g*., a donor), the pressure sensor A1 may communicate with the controller 18 to monitor the pressure within the vein of the blood source.

The cassette includes two valve stations downstream of the source pump P2, with valve V2 being closed to prevent flow through line L5 and valve V1 being open to allow flow through line L6. A portion of the blood is directed through line L7 and a cassette sensor station associated with pressure sensor A3 to the in-process container F3 and the remainder is directed through line L8 toward the centrifuge pump P3, which controls the amount of blood that is directed to the centrifugal separation chamber 36 instead of the in-process container F3. In particular, the flow rate of the source pump P2 is greater than the flow rate of the centrifuge pump P3, with the difference therebetween being equal to the flow rate of blood into the in-process container F3. The flow rates may be selected such that the in-process container F3 is partially or entirely filled with blood at the end of the draw phase.

The blood pumped through line L8 by the centrifuge pump P3 passes through line L9, an air trap 62, and a cassette sensor station associated with pressure sensor A2 (which works in combination with the controller 18 of the fluid processing device 10 to monitor the pressure in the centrifugal separation chamber 36) before reaching the centrifugal separation chamber 36 of the fluid flow circuit 12. The centrifugal separator 16 of the fluid processing device 10 manipulates the centrifugal separation chamber 36 to separate the blood in the centrifugal separation chamber 36 into platelet-rich plasma and packed red blood cells. In one embodiment, the centrifugal separation chamber 36 is rotated nominally at 4,500 rpm, but the particular rotational speed may vary depending on the flow rates of fluids into and out of the centrifugal separation chamber 36.

The packed red blood cells exit the centrifugal separation chamber 36 via line L10 and flow through line L11 into the return container F4. Platelet-rich plasma is drawn out of the centrifugal separation chamber 36 via line L12 by the combined operation of the recirculation and outlet pumps P5 and P4 of the fluid processing device 10. The platelet-rich plasma travels through line L12 until it reaches a junction, which splits into lines L13 and L14. The recirculation pump P5 is associated with line L13 and redirects a portion of the platelet-rich plasma to a junction, where it mixes with blood in line L8 that is being conveyed into the centrifugal separation chamber 36 by the centrifuge pump P3. Recirculating a portion of the platelet-rich plasma into the centrifugal separation chamber 36 with inflowing blood decreases the hematocrit of the blood entering the centrifugal separation chamber 36, which may improve separation efficiency. By such an arrangement, the flow rate of the fluid entering the centrifugal separation chamber 36 is equal to the sum of the flow rates of the centrifuge pump P3 and the recirculation pump P5. As the platelet-rich plasma drawn out of the centrifugal separation chamber 36 into line L13 by the recirculation pump P5 is immediately added back into the centrifugal separation chamber 36, the bulk or net platelet-rich plasma flow rate out of the centrifugal separation chamber 36 is equal to the flow rate of the outlet pump P4.

Line L14 ends at a junction, where it joins with lines L15 and L16. Valve V6 is closed to prevent fluid flow through line L16, thereby directing the separated platelet-rich plasma to the spinning membrane separator 26 via line L15. Before reaching the spinning membrane separator 26, the portion of the platelet-rich plasma conveyed through line L15 passes the spinner inlet sensor M1 and a cassette sensor station associated with pressure sensor A4. The spinner inlet sensor M1 may detect the concentration of platelets in the platelet-rich plasma entering the spinning membrane separator 26, while the pressure sensor A4 may monitor the pressure of the spinning membrane separator 26.

While valve V6 is shown in FIG. 4 as being closed, it may be selectively opened to divert all or a portion of the platelet-rich plasma from line L14 into and through line L16 and to the return container F4, if necessary. An example would be at the start of a procedure when separation is initializing and platelets are not yet exiting the centrifugal separation chamber 36, in which case the fluid conveyed through line L14 by the outlet pump P4 could be diverted to the return container F4.

The spinning membrane separator drive unit 14 of the fluid processing device 10 manipulates the spinning membrane separator 26 to separate the platelet-rich plasma into platelet-poor plasma ("plasma") and platelet concentrate ("platelets"). Plasma is pumped out of the spinning membrane separator 26 via line L17 by the plasma pump P6 of the fluid processing device 10. Valves V5, V6, V8, and V9 are closed to direct the separated plasma along line L18, through valve V4, and into the return container F4 (with the separated red blood cells). On the way to the return container F4, the plasma passes through spinner outlet sensor M2, which may cooperate with the controller 18 to determine one or more characteristics of the plasma, such as the amount of cellular blood components in the plasma and/or whether the plasma is hemolytic and/or lipemic.

The platelet concentrate is conveyed out of the spinning membrane separator 26 via line L19. There is no pump associated with line L19, so instead the flow rate at which the platelets exit the spinning membrane separator 26 is equal to the difference between the flow rates of the outlet pump P4 and plasma pump P6. Valve V8 is closed to prevent fluid flow through the line L20, thereby directing the flow of platelets along line L19, through valve V7, and into the platelet collection container F6. Valve V8 may be selectively opened to allow fluid flow through line L20 and to a junction, where it joins the plasma flowing through line L18 to the return container F4, if necessary.

Depending on the volume of platelets to be collected, the draw stage of FIG. 4 may be repeated, with draw stages being alternated with return stages in which blood from the in-process container F3 is separated in the centrifugal separation chamber 36 while previously collected blood components in the return container F4 are returned to the blood source. During such return stages, the separated red blood cells and platelet-rich plasma may be variously routed through the fluid flow circuit 12, typically with an additional volume of platelets being collected in the platelet collection container F6 after being separated from platelet-poor plasma in the spinning membrane separator 26 (as during the draw stage of FIG. 4. A platelet additive solution from the additive container F7 may be added to the collected platelets in the platelet collection container F6 before ending the procedure.

### IV. Notification System

As described herein, controller 18 may receive signals from various components of the fluid processing device 10 to monitor various aspects of the fluid processing device 10 and characteristics of the fluid and separated fluid components as they flow through the fluid flow circuit 12. If the operation of any of the components, an error condition is present (*e.g.*, air is present in the tubing or after detection of leaks and obstructions in the fluid flow circuit 12.) and/or one or more characteristics of the fluid or the separated fluid components (*e.g*., fluid pressure, hematocrit, platelet concentration, and/or volume) is outside of an acceptable hard-coded range or value, then the controller can provide an alert and/or notification to the operator. In an embodiment, the alert can be an audible alert. For instance, the audible alert can be a chime, alarm, ding, voice recording, or other suitable audible alert. The device 10 can include a speaker to provide the audible alert. In some embodiments, the alert can be a visual alert. For instance, the controller 18 can instruct the device 10 to illuminate a status light, the operator interface (if present) to display a visual alert, such as an alpha-numeric message, or can instruct the device 10 to display any other suitable visual alert. In an example, device 10 can be associated with an external device wirelessly or via a wire connection and the controller 18 can be configured to provide the alert and/or notification via the external device. The external device can be, but is not limited to, a panel configured to monitor all active instruments of a system (including device 10). The external device may include speakers, lights, and/or a screen. The alert can be an audible alert and/or a visual alert. For instance, device 10 can be configured to provide real-time information through a data management system such as, but not limited to the Fresenius DXT System. The real-time information from the device 10 can be monitored remotely on the external device.

In addition to being configured to provide an alert and/or notification based on hard-coded values, the controller 18 is configured to provide an "intelligent" alert and/or notification based on a monitored event during a blood processing procedure. For instance, controller 18 can provide an alert and/or notification that corresponds to dynamic events during the procedure rather than only alerting an operator when a particular value is detected, thus resulting in a more personalized and efficient procedure.

Similar to providing an alert based on a hard-coded value, controller 18 is configured to provide an "intelligent" alert and/or notification using the various sensors of the device 10. The controller 18 can be configured to receive information from the various sensors of the system, store the information, and analyze the information to determine when and how to provide an alert and/or notification. Additionally, the controller 18 can be configured to record the actions and operation of the device 10 and use this information to determine when and how to provide an alert and/or notification. In some embodiments, the controller 18 includes an on-board procedure estimator. The on-board procedure estimator can be configured to determine procedure results based on system settings. For instance, the on-board procedure estimator can determine procedure results based on parameters entered by the operator before starting the procedure, on adjustments made by the controller 18 mid-procedure, and/or by operator-entered adjustments mid-procedure. For instance, the on-board procedure estimator can determine procedure results by taking into account factors such as air detection/purges, centrifuge line blockages that require saline to assist in clearing the line, procedure efficiency due to flow rates (which may be controller 18 or operator adjusted), or operator-initiated events such as saline infusion mid procedure (*e.g*., due to a citrate reaction or for preventing blood coagulation during extended pauses/stoppages). Additionally, the controller 18 can include an optimizer. The optimizer can be configured to use the output(s) of the on-board estimator and inputs not used by the estimator, such as, but not limited to, blood type and/or donor/donation history, to determine the best procedure configuration and targets.

In an example, the monitored event can include an optimal point in the procedure where a parameter can be changed. In some embodiments, an operator can manually adjust the parameters of a procedure during the procedure. The controller 18 can be configured to determine a safe and/or optimal moment during the procedure at which a parameter can be adjusted. By utilizing the on-board procedure estimator of the controller 18 and information from the various sensors of the device 10, it is possible to predict the impact of changing procedure inputs such as target dose (*i.e.*, changing from a double platelet dose to a triple platelet dose or collecting a concurrent plasma product), target product amount/volume, donor parameters (*i.e*., pre-count), or procedure settings (*i.e*., CIR rate), as well as any other suitable procedure input. The optimal time can be any time in the procedure outside of a time (*e.g*., before or after) where changing the parameter would not be recommended and, therefore, would be considered suboptimal. A suboptimal time can be a point in the procedure where changing the parameter could be deemed unsafe for the patient/donor. In some embodiments, the controller 18 can alert the operator with a consistent amount of time remaining before the point in the procedure when changing the parameter would be unsafe for the donor/patient. For example, the controller 18 can be configured to alert the operator with 5 minutes, 10 minutes, or any other suitable amount of time before it would be unsafe to adjust a parameter.

In some embodiments, controller 18 can optionally be configured to lock a parameter (*i.e*., disallow any adjustments to a parameter) when the optimal point in the procedure for the parameter to be changed is not present. For instance, the controller 18 can be configured to lock a parameter when the optimal point in the procedure for the parameter to be changed has passed. For example, an operator would not be able to change the parameters to finish the procedure early if the optimal point to adjust those parameters that would result in finishing early had passed. Additionally, in some embodiments, an operator (or an administrator or supervisor) can select which parameters to lock after the optimal point to adjust the parameter has passed. For instance, before starting a procedure, an operator can select a setting via the user interface (if present) to configure the controller 18 to lock a parameter after the optimal point to change that parameter has passed. By configuring the controller 18 to lock parameters after their respective optimal time to be adjusted has passed, an operator does not need to determine whether it is unsafe to change a parameter at a particular time for the patient, thus easing the cognitive load on the operator.

In some embodiments, the monitored event can include an amount of time remaining in the blood processing procedure. In an example, the controller 18 can be configured to provide an alert and/or notification with an amount of time remaining in the procedure being carried out by device 10. The amount of time can be consistent between the procedures carried out by the device 10. For instance, the controller 18 can provide an alert and/or notification with 5 minutes, 10 minutes, or any other suitable amount of time remaining in the procedure.

Typically, a device may provide an alert that a procedure is close completion based on volume, yet this results in alerts being provided at inconsistent times because of the variable flow rates between procedures. By configuring the controller 18 to monitor the procedure, controller 18 can analyze the information from the various sensors of the device 10 (*e.g*., flow rate) to provide an alert and/or notification at an amount of time remaining in the procedure. In this manner, the controller 18 can alert the operator with a consistent amount of time remaining in the procedure, regardless of the procedure, providing the operator with a consistent amount of time to prepare any subsequent post-procedure materials (if necessary). For instance, the operator is alerted to gather any supplies needed for donor/patient disconnect/post-processing and to begin taking donor/patient post-procedure vitals. By being alerted at a consistent time at the end of procedures, operators are also able to cycle through donors/patients more efficiently, therefore allowing donation centers/treatment centers to receive more donors/patients.

Additionally, in some embodiments, the monitored event can include an amount of time remaining in a procedure until a solution container associated with the disposable circuit 12 is empty. In particular, the controller 18 can be configured to provide an alert and/or notification when a particular amount of time remains until a solution container (*e.g*., F1, F2, and/or F7) associated with the disposable circuit 12 is empty. In some examples, one or more solution containers can contain any suitable solution, such as, but not limited to an additive solution (*e.g*., PAS), saline, and anticoagulant (*e.g*., anticoagulant citrate dextrose (ACD)). Additionally, the one or more solution containers can be monitored using the volume measurement systems W1-W6. As described herein, the controller 18 can be configured to monitor the rate of the procedure. Using the rate of the procedure and information from the volume measurement systems W1-W6, the on-board procedure estimator of the controller 18 can determine if a particular solution container contains enough solution for the remainder of the procedure. The controller 18 can be configured to provide an alert and/or a notification at a consistent time, such as 5 minutes, 10 minutes, or any other suitable time until a container is empty. By providing an alert with a consistent amount of time remaining until a solution container is empty, an operator can be notified to check the solution in the container and to gather additional containers to replace any container that is nearly empty. The operator can then replace the container (after placing the system in a safe configuration to replace the container) to ensure minimal down time, thus increasing efficiency of the procedure. For example, the controller 18 can be configured to notify an operator if collection containers containing PAS or saline (used for reinfusion/post-collection) are nearing empty or if the amount of anticoagulant solution (*e.g*., ACD) is sufficient to complete the procedure.

Additionally, in some embodiments, the controller 18 can be configured to provide an alert and/or notification when a particular amount of time in a particular step of a blood processing procedure is remaining. For instance, the controller 18 can be configured to monitor the processing rate of the various steps of a blood processing procedure. Based on the monitored rate, the controller 18 can determine how much time is remaining during the step. The controller 18 can then provide an alert and/or notification when a particular amount of time remains during a step of the procedure. For example, the controller 18 can provide an alert and/or notification with 5 minutes left during a step, 10 minutes left during a step, or any other suitable time left during a step of the procedure. The controller 18 can be configured to provide an alert and/or notification during various steps of a blood processing procedure, such as, but not limited to, whole blood collection, separation of whole blood into blood products, collection of separated blood products, and/or any other suitable step during a blood processing procedure.

Optionally, in some embodiments, the controller 18 can be configured to provide an alert and/or notification after a period of time from the start of the procedure or at set intervals during the procedure. In some embodiments, the operator (or administrator or supervisor) can set the controller to provide the alert and/or notification at a selected time or interval of time. For example, the operator (or administrator or supervisor) can set the controller 18 to provide an alert and/or notification after a certain period of time to check-in on the donor/patient. This can be done to remind the operator to assess how the donor/patient is feeling, for example, after the donor/patient reports being cold or uncomfortable, or after having a mild citrate reaction.

Furthermore, in some embodiments, the controller 18 can be configured to provide an alert and/or notification after certain events in the procedure or when a certain event has not occurred in the procedure. For example, the controller 18 can provide an alert and/or notification if the operator has not entered updated donor parameters from pre-samples after a point at which the information should have been entered into the system (*i.e*., inputted into the controller 18). The controller 18 can provide an alert and/or notification after any suitable event during a procedure. In some instances, the operator (or administrator or supervisor) can pre-select the event that affects the controller 18 to provide an alert and/or notification.

In some embodiments, the monitored event can include the blood processing procedure approaching a selected target product amount. For example, the controller 18 can be configured to provide an alert and/or notification to the operator when the targeted blood product approaches a pre-selected/pre-determined targeted amount. For instance, the controller 18 can provide this alert and/or notification based on an amount/volume of targeted blood product collected or on the amount of time remaining in the procedure. In a particular example, the controller 18 can be configured to provide the alert and/or notification based on the amount/volume of a targeted blood product collected, which can be determined by the on-board estimator with factors, such as the volume processed and time remaining in the procedure. The targeted blood product can be, but is not limited to, an amount of platelets, volume of plasma, volume of red blood cells, and/or other suitable blood product. After providing an alert and/or notification that the procedure is approaching a target product amount, using information from the on-board procedure estimator and optimizer, the operator can suggest a more optimum procedure to a donor (if a more optimum procedure is available) or if the donor would be willing to stay longer. For instance, this may include an adjustment to the procedure, such as, but not limited to, collecting an additional blood product or changing a procedure parameter (*e.g*., flow rate). This can provide an increase in the overall products collected or more valuable products for the donation center/treatment center.

In some embodiments, the monitored event can include an adjustment to a pre-determined parameter in the blood processing procedure. As described herein, the controller 18 is configured to instruct a device 10 to carry out a blood processing procedure. For example, the controller 18 is configured to instruct the device to open valves, to operate pumps to affect the flow of fluid through a disposable circuit 12, to operate the separation chamber, etc. Before starting a blood processing procedure, the operator (or administrator or supervisor) may input pre-determined parameters to the controller 18 and/or the controller may pre-determine parameters based on the procedure selected. For example, the pre-determined parameters can include, but are not limited to, target product amount, flow rate, pressure, time of procedure, target volume of whole blood to process, spinner speed, anticoagulant ratio, citrate infusion rate, and cuff pressure. During the procedure, the controller 18 can be configured to instruct the device 10 to take an action in response to information received from one or more of the sensors or in response to an error condition. In some instances, the action can be to adjust one or more of the pre-determined parameters to continue the procedure. The controller 18 is configured to provide an alert and/or notification when the controller 18 makes an adjustment to the one or more pre-determined parameter. By alerting the operator that the controller 18 has adjusted a pre-determined parameter, the operator can check if the action was necessary or can override the action.

As an illustrative example, the controller 18 can be configured to detect a vein occlusion event and in response can be configured to raise the cuff pressure and/or lower the draw rate of the procedure. In this instance, the controller 18 is configured to provide an alert and/or notification after the responsive action. Accordingly, the operator is notified that the controller 18 instructed device 10 to perform an action to maintain good flow. By providing the alert and/or notification, the operator can then remind the donor to squeeze or can override the action if the vein occlusion was not a re-occurring issue (*i.e*., the donor moved their arm).

In another example, the controller 18 can provide an alert and/or a notification to the operator when an adjustment is made to the time of the procedure and/or to the amount/volume of fluid to be processed (*e.g*., whole blood). For example, the time of the procedure may be extended or shortened. As described herein, in some instances, an operator can make manual adjustments to certain parameters during the procedure. For example, the operator can change donor parameters such as pre-count or red cell content, procedure targets or settings within the procedure (*i.e*., AC ratio/citrate infusion rate). Additionally, the controller 18 can instruct the device 10 to take an action such as adjust a particular parameter in order to complete the procedure. In these instances, although the time and or volume of fluid to be processed is not directly adjusted, adjusting other parameters or settings can indirectly lead the controller 18 to adjust the time and or volume of fluid to be processed. As such, the controller 18 is configured to provide an alert and/or notification to the operator that the time has been shortened or extended and/or that the volume of fluid (*e.g*., whole blood) to be processed has been increased or decreased.

In some embodiments, the monitored event can include a device performance event. In this instance, the controller 18 is configured to provide an alert and/or notification to the operator when the device 10 detects an issue with the procedure, such as an error condition as described herein. For example, using the various sensors of the device 10, the device 10 can detect vein occlusions, poor interface control (*i.e*., spillovers), the presence of lipemic plasma, and other possible procedure issues. In the instance lipemic plasma is detected, the system may not be able to continue the collection procedure or may perform the collection poorly unless an adjustment is made (*e.g*., an adjustment to the configuration of the optical measurement system including light source 50 and light detector 52). In the case of a device performance event, the controller 18 can be configured to provide the alert and/or notification as a gentle reminder to the operator so as to not alarm the donor/patient. In other words, the alert and/or alarm can be gentler than the other alerts and or notifications described herein. For example, the alert and or notification can be a chime. The operator can check the device 10 and determine whether to continue, pause, or terminate the procedure.

There are additional aspects to the devices and methods described herein including, without limitation the following aspects.

Aspect 1. A blood processing system including a reusable separation device, wherein the separation device includes a separator; a disposable fluid circuit including a separation chamber and a plurality of containers, wherein the disposable fluid circuit configured to be associated with the reusable separation device; and wherein the reusable separation device includes a controller configured to monitor a blood processing procedure and to provide one or more notifications based on a monitored event during the blood processing procedure.

Aspect 2. The system of Aspect 1, wherein the one or more notifications is an audible notification or a visual notification.

Aspect 3. The system of any one of Aspects 1-2, wherein the monitored event includes an optimal point in the procedure where a parameter can be changed.

Aspect 4. The system of Aspect 3, wherein the controller is configured to lock the parameter after the optimal point in the procedure where a parameter can be changed has passed.

Aspect 5. The system of any one of Aspects 3-4, wherein the parameter includes target dose, target product amount, donor parameters, and/or procedure settings.

Aspect 6. The system of any one of Aspects 1-5, wherein the monitored event includes an amount of time remaining in the blood processing procedure.

Aspect 7. The system of Aspect 6, wherein the controller is configured to determine the amount of time remaining in the blood processing procedure based on a processing rate of the blood processing procedure.

Aspect 8. The system of any one of Aspects 1-7, wherein the monitored event includes the blood processing procedure approaching a selected target product amount.

Aspect 9. The system of any one of Aspect 1-8, wherein the monitored event includes an amount of time remaining in a procedure until a solution container associated with the disposable circuit is empty.

Aspect 10. The system of Aspect 9, wherein the controller is configured to determine the amount of time remaining in the procedure based on a processing rate of the blood processing procedure.

Aspect 11. The system of any one of Aspects 1-10, wherein the monitored event includes an adjustment to a pre-determined parameter in the procedure.

Aspect 12. The system of Aspect 11, wherein the adjustment includes extending or shortening a pre-determined time of the procedure.

Aspect 13. The system of Aspect 11, wherein the adjustment includes increasing or decreasing the volume of fluid to be processed.

Aspect 14. The system of any one of Aspects 1-13, wherein the monitored event includes a device performance event.

Aspect 15. The system of Aspect 14, wherein the device performance event includes vein occlusion, interface control between separated blood components, and/or presence of lipemic plasma.

Aspect 16. The system of any one of Aspects 1-15, wherein the controller is configured to provide a notification after a pre-determined period of time.

Aspect 17. A blood processing device including a separator; and a controller configured to monitor a blood processing procedure and to provide one or more notifications based on a monitored event during the blood processing procedure.

Aspect 18. The device of Aspect 17, wherein the device is configured to be associated with a disposable fluid circuit including a separation chamber and a plurality of containers.

Aspect 19. The device of any one of Aspects 17-18, wherein the one or more notifications is an audible notification or a visual notification.

Aspect 20. The device of any one of Aspects 17-19, wherein the monitored event includes an optimal point in the procedure where a parameter can be changed.

Aspect 21. The device of Aspect 20, wherein the controller is configured to lock the parameter after the optimal point in the procedure where a parameter can be changed has passed.

Aspect 22. The device of any one of Aspects 20-21, wherein the parameter includes target dose, target product amount, donor parameters, and/or procedure settings.

Aspect 23. The device of any one of Aspects 17-22, wherein the monitored event includes an amount of time remaining in the blood processing procedure.

Aspect 24. The device of Aspect 23, wherein the controller is configured to determine the amount of time remaining in the blood processing procedure based on a processing rate of the blood processing procedure.

Aspect 25. The device of any one of Aspects 17-24, wherein the monitored event includes the blood processing procedure approaching a selected target product amount.

Aspect 26. The device of any one of Aspects 18-25, wherein the monitored event includes an amount of time remaining in the procedure until a solution within one of the plurality of containers of the disposable fluid circuit is empty.

Aspect 27. The device of Aspect 26, wherein the controller is configured to determine the amount of time remaining in the procedure based on a processing rate of the blood processing procedure.

Aspect 28. The device of any one of Aspects 17-27, wherein the monitored event includes an adjustment to a pre-determined parameter in the procedure.

Aspect 29. The device of Aspect 28, wherein the adjustment includes extending or shortening a pre-determined the time of the procedure.

Aspect 30. The device of Aspect 28, wherein the adjustment includes increasing or decreasing the volume of fluid to be processed.

Aspect 31. The device of any one of Aspects 17-30, wherein the monitored event includes a device performance event.

Aspect 32. The device of Aspect 31, wherein the device performance event includes vein occlusion, interface control between separated blood components, and/or presence of lipemic plasma.

Aspect 33. The device of any one of Aspects 17-32, wherein the controller is configured to provide a notification after a pre-determined period of time.

It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

## Claims

1. A blood processing system comprising:
a reusable separation device, wherein the separation device includes a separator;
a disposable fluid circuit including a separation chamber and a plurality of containers, wherein the disposable fluid circuit configured to be associated with the reusable separation device; and
wherein the reusable separation device includes a controller configured to monitor a blood processing procedure and to provide one or more notifications, preferably an audible notification or a visual notification, based on a monitored event during the blood processing procedure.

2. The system of any one of claims 1, wherein the monitored event includes an optimal point in the procedure where a parameter can be changed.

3. The system of any one of claims 1-2, wherein the monitored event includes an amount of time remaining in the blood processing procedure.

4. The system of any one of claims 1-3, wherein the monitored event includes the blood processing procedure approaching a selected target product amount.

5. The system of any one of claim 1-4, wherein the monitored event includes an amount of time remaining in a procedure until a solution container associated with the disposable circuit is empty.

6. The system of any one of claims 1-5, wherein the monitored event includes an adjustment to a pre-determined parameter in the procedure.

7. The system of any one of claims 1-6, wherein the monitored event includes a device performance event.

8. A blood processing device comprising:
a separator; and
a controller configured to monitor a blood processing procedure and to provide one or more notifications, preferably an audible notification or a visual notification, based on a monitored event during the blood processing procedure.

9. The device of claim 8, wherein the device is configured to be associated with a disposable fluid circuit including a separation chamber and a plurality of containers.

10. The device of any one of claims 8-9, wherein the monitored event includes an optimal point in the procedure where a parameter can be changed.

11. The device of any one of claims 8-10, wherein the monitored event includes an amount of time remaining in the blood processing procedure.

12. The device of any one of claims 8-11, wherein the monitored event includes the blood processing procedure approaching a selected target product amount.

13. The device of any one of claims 8-12, wherein the monitored event includes an amount of time remaining in the procedure until a solution within one of the plurality of containers of the disposable fluid circuit is empty.

14. The device of any one of claims 8-13, wherein the monitored event includes an adjustment to a pre-determined parameter in the procedure.

15. The device of any one of claims 8-14, wherein the monitored event includes a device performance event.
